# EUROPEAN PATENT APPLICATION

(11) **EP 1 637 136 A1**
(43) Date of publication of application: **22.03.2006**
(21) Application number: 04735099.6
(22) Date of filing: 27.05.2004
(51) Int. Cl.: A61K 31/34, C07D 493/04, A61P 25/02, A61P 9/02

(54) **COMPOSITION HAVING AUTONOMIC NERVE MODULATING ACTIVITY AND METHOD OF USE THEREOF**

(30) Priority: 27.05.2003 JP 2003149361
(71) Applicant: SUNTORY LIMITED, Osaka-shi, Osaka 530-8203 (JP)
(72) Inventor: MORITANI, Toshio, Kyoto-shi, Kyoto 612-8034 (JP); KISO, Yoshinobu, 5670872 (JP); ONO, Yoshiko, Osaka-shi, Osaka 533-0022 (JP)
(74) Representative: Stoner, Gerard Patrick
(86) International application number: PCT/JP2004/007641
(87) International publication number: WO 2004/105749

(57) **Abstract**

A composition having autonomic nerve function regulating action, comprising sesamin and/or episesamin as an active ingredient, is provided.

## Description

### TECHNICAL FIELD

The present invention relates to a composition possessing autonomic nerve function regulating action and the use thereof.

### BACKGROUND ART

A living body has an autonomic nerve system, an endocrine system, and an immune system, which function to maintain the homeostasis of the living body. Among them, the autonomic nerve system functions relatively independently of the brain and functions automatically, independently of the will, and thus is named for this property. The autonomic nerve system mainly regulates functions of internal organs. The autonomic nerve system includes two systems, a sympathetic nerve system and a parasympathetic nerve system. The autonomic nerve system is controlled by the balance between both the systems. Specifically, when the body is in an active state, the activity of the sympathetic nerves is dominant and, consequently, the whole body is brought to a state of tension. On the other hand, when the activity of the parasympathetic nerves is dominant, the tension of the body is eliminated and the body is relaxed.

It is known that, when a stress-derived disturbance of the activity of the autonomic nerves continues for a long period of time, various types of physical illness occur. The disturbance of the activity of the autonomic nerves refers to such a state that the activity of the sympathetic nerves is dominant or that the activity of the parasympathetic nerves is significant. In particular, in today's society, health problems caused by continuance of the sympathetic nerve activity-dominant state is acknowledged as a problem. The sympathetic nerve activity-dominant state is caused when the activity of the sympathetic nerves has been increased, when the activity of the parasympathetic nerve system has been lowered without a change in the activity of the sympathetic nerves, or when the activity of the sympathetic nerves has been increased with a lowering in the activity of the parasympathetic nerves.

When the balance of the autonomic nerves is disturbed as a result of an increase in the activity of the sympathetic nerves, the secretion of adrenomedullary hormones is accelerated, resulting in an increase in heart rate and blood pressure, bronchiolectasis, inhibition of motion and secretion of the intestine, an increase in glucose metabolism, mydriasis, piloerection, vasoconstriction of skin and internal organs, and angiectasia of the skeletal muscle (Iwanami Koza "Gendai Igaku No Kiso (Introduction to modern medicine)", Vol. 4, Toshio Hagiwara and Seiichiro Tarui, eds, Seitai No Chosetsu Shisutemu (Regulation system in living body), 1999). Therefore, increased activity of the sympathetic nerves causes health problems such as hypertension, hyperglycemia, lowered skin bloodstream, and deteriorated immune functions. When the activity of the parasympathetic nerves has been increased, chronic diarrhea occurs. In recent years, it has been pointed out that lowered activity of the parasympathetic nerves is an important risk factor of coronary cardiac diseases and sudden death.

In addition to the disturbance of the balance between the activity of the sympathetic nerves and the activity of the parasympathetic nerves, in some cases, both the activity of the sympathetic nerves and the activity of the parasympathetic nerves are lowered. For example, it is reported that, for diabetics, in general, the activity of the sympathetic nerves and the activity of the parasympathetic nerves are simultaneously lowered to levels lower than those for healthy people (Yamasaki Y et al., Diabetes Res. 17: 73-80 (1991), Bellavere F et al., Diabetes, 41: 433-40 (1992)). Further, dysautonomia is causative of diseases including sudden death, silent myocardial infarction, and asymptomatic hypoglycemia (Nihon Rinsho (Japan Clinics), Vol. 60, first issue 10, 2002).

At the present time, these health problems are symptomatically treated for each symptom or disease, and a method in which the activity of the autonomic nerves per se, i.e., the underlying cause of the above symptoms or diseases, is controlled, is not adopted. The symptomatic treatment can temporarily ameliorate the clinical state per se. However, when the drug is discontinued, the symptom or disease reoccurs. On the other hand, when the drug is continued, even though one disease could be ameliorated, other diseases derived from the underlying cause of the symptom or the disease, i.e., the disturbance of autonomic nerves and the lowering in the activity of autonomic nerves are highly likely to develop. Therefore, pharmaceutical products or health foods, which can control the underlying cause of the symptom or disease, i.e., the activity of the autonomic nerves per se, have been desired. That is, if compounds, which can return the sympathetic nerve activity-dominant state, the parasympathetic nerve activity-dominant state, or the totally increased or lowered activity of the autonomic nerves to the original normal state, by inhibiting the activity of the sympathetic nerves or parasympathetic nerves increased by some cause, or increasing the activity of the sympathetic nerves or parasympathetic nerves lowered by some cause, could be developed, then the value of such compounds is very high also from the viewpoint of preventing various diseases.

Smoking, exercise, diabetes, heart diseases, obesity and the like may be mentioned as causes of the disturbance of the activity of the autonomic nerves. It is reported that smoking causes a lowering in the activity of parasympathetic nerves and a relative increase in the activity of sympathetic nerves which are an important risk factor of ischemic heart diseases and sudden death (Akselrod et al., Science 213: 220-22 (1981), G.E. Billman et al., Circulation 80: 874-80 (1989)). Further, the disturbance of the autonomic nerves is also caused by stress, and many people report poor a physical condition of unknown cause.

Diabetes and obesity, hard exercise, aging and the like may be mentioned as causes of totally lowered activity of the autonomic nerves. Regarding diabetics, it is reported that both the activity of the sympathetic nerves and the activity of the parasympathetic nerves are lowered and that, in particular in an early stage, the lowering in the activity of the parasympathetic nerves occurs in advance of the lowering in the activity of the sympathetic nerves (Yamasaki Y et al., Diabetes Res. 17: 73-80 (1991)), and that, in addition to the lowering in activity, a lowering in response of the activity of the sympathetic nerves and the activity of the parasympathetic nerves is observed (Shintani et al., Jpn. J Electrocardiol., 18: 40-45 (1998)). For fat persons, it is known that, in addition to a lowering in the activity of the parasympathetic nerves, a lowering in the activity of the sympathetic nerves is observed and, in addition, the response of the activity of the autonomic nerves is lowered (Peterson HR et al., N Engl J Med. 318: 1077-83 (1988), Matsumoto T et al., Int J Obes Relat Metab Disord. 23: 793-800 (1999)). It is also reported that the activity of the autonomic nerves is lowered by aging (Oida E et al., J Gerontol Med Sci. 54: 219-224 (1999)).

The autonomic nerve system is a nerve system which a person cannot control by his or her own will and functions to maintain life activity such as breathing, circulation, digestion, metabolism, excretion, and body temperature regulating function and, further, is also deeply involved in the endocrine system and the immune system. A disturbance of the balance of the autonomic nerves having the above various functions causes various disorders both physically and mentally. Symptoms which are likely to be caused by the disturbance of the autonomic nerves include shortness of breathing (tachypnea), palpitation, stiffness in the shoulder, headache, vertigo, sense of uneasiness, anorexia, malaise, and insomnia. In this connection, it should be particularly noted that there is a report on climacteric age to the effect that 75% of climacteric disorders is caused by autonomic nerve imbalance. Painful symptoms such as hot flash, glow, psychroesthesia, and acute sweating in climacteric disorders are also caused by body temperature regulation disorder attributable to the disturbance of the activity of the autonomic nerves. The activity of the sympathetic nerves is also involved in heat production, and the sympathetic nerves act so as to burn fat. Therefore, lowered activity of the sympathetic nerves is also a cause of energy metabolic disturbance.

Thus, as the lowering or disturbance of the activity of the autonomic nerves causes various physical and mental disorders, the development of compounds, that have excellent adaptation to pharmaceutical products and foods which can regulate the activity of the autonomic nerves and are less likely to entail side effects, has been strongly desired.

Sesamin is a kind of major lignan compound contained in sesame and is contained in an amount of about 0.5 to 1% in sesames. Sesamin has Δ5 unsaturated enzyme inhibitory action (S. Shimizu et al., J. Am. Oil Chem. Soc., 66, 237-241 (1989), and S. Shimizu et al., Lipid, 26, 512 (1991)), antioxidative action (Japanese Unexamined Patent Publication (Kokai) No. 5(1993)-051388 and Japanese Patent Application No. 11(1999)-327924), lipid peroxide level increase inhibitory effect in an experimental breast cancer development model (N. Hirose et al., Anticancer Research, 12, 1259-1266 (1992)), vitamin E protective action (K. Yamashita et al., J. Nutr., 122, 2440 (1992), K. Yamashita et al., Lipid, 30, 1019 (1995), and A. Kamal-Eldin et al., Lipids, 30, 499 (1995)), DHA protective action (K. Yamashita et al., Biofactors, 11, 11-13. (2000)), and inhibitory effect against blood lipid peroxide level increase involved in hard excercise (T. Ikeda, et al., Int. J. Sports Med., (2003) in print).

Japanese Unexamined Patent Publication (Kokai) No. 8(1996)-268887 discloses antihypertensive action of sesamin and reports that sesamin has antihypertensive action in a DOCA salt loading hypertension model and a renal-clip renal hypertension model (Y. Matsumura et al., Biol. Pharm. Bull., 18, 1016 (1995), and S. Kita et al., Biol. Pharm. Bull., 18, 1283 (1995)). Further, inhibitory action for a stroke-prone spontaneously hypertensive rat (SHR-SP) is disclosed (Y. Matsumura et al., Biol. Pharm. Bull., 21, 469 (1998)).

The relationship between hypertension and active oxygen (particularly superoxide) is pointed out (X. Zhang et al., Am. J. Physiol., 258, 497 (1990), and M. Torii et al., Ipn. Heart J., 30, 589 (1989)), and it is reported that the intravenous administration of superoxide dismutase (SOD) to SHR rats has antihypertensive action (K. Nakazono et al., Proc. Natl. Acad. Sci. U.S.A., 88, 10045 (1991)).

Further, a psychotropic action of sesamin is disclosed in US Patent No. 4427694. Specifically, US Patent No. 4427694 discloses that sesamin has tranquilizing effect, antidepressant effect, and anticonvulsant effect and can alleviation of alcohol or tobacco withdrawal symptoms. All of mental symptoms indicated here involve action through the central nervous system (Kinya Kuriyama and Tomohiro Matsuda, editors and authors, Nou To Shinkei No Yakuri (Pharmacology of brain and nerve), Kinpodo (1984), Katsura M et al., J Biol Chem. 277: 7979-7988 (2002), and Aoki et al., Hoken No Kagaku (Health science), 43: 217-226 (2001)), but not action against an autonomic nerve system.

Despite the fact that various actions of sesamin have been reported, up to now, the action and regulatory action of sesamin against the activity of the autonomic nerves have not been examined and reported.

### DISCLOSURE OF THE INVENTION

An object of the present invention is to provide a safe composition which can act directly on autonomic nerves and can ameliorate the lowered activity of the autonomic nerves and regulate the activity of the autonomic nerves.

With a view to attaining the above object, the present inventors considered that traditional pharmaceutical products or health foods may have autonomic nerve function regulating actions.

Methods for evaluating the activity of the autonomic nerves include biological and physical measurement methods such as electrocardiographic measurement, sphygmomanometry, galvanic skin reflex measurement, and pupil diameter measurement and biochemical measurement methods such as blood catechol amine concentration measurement. Among them, the analysis of heart rate variability with an electrocardiograph is the most commonly used method for measuring activity of the parasympathetic nerves, the activity balance between the sympathetic nerve and the parasympathetic nerve, and the activity of the autonomic nerves (Shindenzu R-R Kankaku Hendo No Supekutoru Kaiseki (Spectral analysis of electrocardiogram R-R interval fluctuation), Jiritsu Shinkei Kino Kensa (Autonomic nerve function inspection), Vol. 2, edited by Japan Society of Neurovegetative Research, p. 57-64, Bunkodo (1995), Ewing DJ et al., Br Hesrt J. 65: 239-244 (1991), and Heart rate variability: standards of measurement, physiological interpretation and clinical use. Circulation, 93: 1043-1065 (1996)).

Accordingly, the present inventors have made extensive and intensive studies, by heart rate variation power spectral analysis, on the action of various foods, food ingredients, and traditional pharmaceutical products against the disturbance of the activity of the autonomic nerves caused by smoking and the total activity of the autonomic nerves and, as a result, have clarified that sesamin, a kind of sesame lignan, and/or its analogues have regulatory action against the disturbance of the activity of the autonomic nerves and ameliorating action against the lowered activity of the autonomic nerves, which has led to the completion of the present invention.

According to the present invention, there is provided an agent having autonomic nerve function regulating action, comprising sesamin and/or episesamin as an active ingredient.

For example, the present invention provides an agent having autonomic nerve function regulating action for use in amelioration in lowered activity of the autonomic nerves, which comprises sesamin and/or episesamin as an active ingredient.

For example, the present invention provides an agent having autonomic nerve function regulating action for use in the prevention, amelioration, or alleviation in the disturbance or totally lowered activity of the autonomic nerves, and symptoms attributable thereto, which comprises sesamin and/or episesamin as an active ingredient.

The autonomic nerve function regulating action is, for example, to inhibit an increase in the activity of sympathetic nerves, or to increase the activity of parasympathetic nerves, or to inhibit a lowering in the activity of parasympathetic nerves, or to ameliorate the lowered activity of autonomic nerves. The above agent is preferably in the form of a food or a drink or a pharmaceutical product.

According to the present invention, there is also provided a method for using the above agent having autonomic nerve function regulating action, said method comprising the step of ingesting sesamin and/or episesamin to regulate the disturbance of the autonomic nerves or to ameliorate the lowered activity of the autonomic nerves, whereby symptoms attributable to the disturbance or lowered activity of the autonomic nerves are prevented, ameliorated, or alleviated.

Further, according to the present invention, there is provided a method for inhibiting an increase in the activity of sympathetic nerves, comprising the step of ingesting sesamin and/or episesamin.

Furthermore, according to the present invention, there is provided a method for increasing the activity of parasympathetic nerves, comprising the step of ingesting sesamin and/or episesamin.

Furthermore, according to the present invention, there is provided a method for inhibiting a lowering in the activity of parasympathetic nerves, comprising the step of ingesting sesamin and/or episesamin.

Furthermore, according to the present invention, there is provided a method for ameliorating totally lowered activity of autonomic nerves, comprising the step of ingesting sesamin and/or episesamin.

Furthermore, according to the present invention, there is provided a method for regulating the disturbance of the activity of autonomic nerves, comprising the step of ingesting sesamin and/or episesamin.

Furthermore, according to the present invention, there is provided a method for ameliorating lowered activity of autonomic nerves, comprising the step of ingesting sesamin and/or episesamin.

Furthermore, according to the present invention, there is provided an inhibitor for inhibiting the prolongation of a.QT interval, comprising sesamin and/or episesamin as an active ingredient.

Furthermore, according to the present invention, there is provided a method for inhibiting the prolongation of a QT interval, comprising the step of ingesting sesamin and/or episesamin.

### BRIEF DESCRIPTION OF THE DRAWINGS

Fig. 1 is a diagram showing inhibitory effect of sesamin against a lowering in the activity of parasympathetic nerves caused by smoking;
Fig. 2 is a diagram showing inhibitory effect of sesamin against prolongation of QT interval caused by smoking; and
Fig. 3 is a diagram showing the effect of ameliorating lowered activity of autonomic nerves attained by sesamin.

### BEST MODE FOR CARRYING OUT THE INVENTION

The present invention will be described in more detail.

The rhythm of heart beats, that is, the interval of heart beats (R-R interval), always varies. This R-R interval variation is called "heart rate variability." This heart rate variability is caused by access of the autonomic nerves to the heart. Therefore, the activity state of the autonomic nerves can be evaluated by monitoring the heart rate variability. The heart rate variability provided from the electrocardiogram is processed by fast Fourier transform to perform a frequency analysis for the provision of a heart rate variability power spectrum. In the power spectrum thus obtained, the low frequency range (low frequency, 0.04 to 0.15 Hz) indicates sympathetic nerve activity and parasympathetic nerve activity, and the high frequency range (high frequency, 0.15 to 0.4 Hz) indicates parasympathetic nerve activity. The balance therebetween can be expressed by low frequency/high frequency (LF/HF) ratio. When the activity of the sympathetic nerve becomes dominant, this value is increased. On the other hand, when the parasympathetic nerve activity becomes dominant, this value is decreased. That is, the heart rate variability power spectral analysis is useful as a method for quantitatively determining the parasympathetic nerve activity and the sympathetic nerve activity/parasympathetic nerve activity balance in a noninvasive manner. Further, a very small change in autonomic nerve activity which is not observed as a variation in blood catechol amine level as a mediator of the autonomic nerve activity can also be detected. In addition, at the present time, a heart rate variability is only means which can realize the measurement of the parasympathetic nerve activity. Therefore, this method is a useful method for the evaluation of the autonomic nerve activity and is widely utilized (Heart rate variability: standards of measurement, physiological interpretation and clinical use. Circulation, 93: 1043-1065 (1996), and Nishimura et al., Rinsho Kensa (Clinical examination), 35: 585-590 (1991)). Further, the inhibition of the activity of the parasympathetic nerves is a risk factor of cardiac muscle-derived sudden death and arrhythmia and is also a technique indispensable for clinical diagnosis.

QT interval reflects the repolarization of the ventricular muscle, that is, the duration of the intracellular action potential, and is also an important measure which reflects the refractory period of the ventricular muscle. The repolarization phase of the cardiac muscle responds very sensitively to various pathologic factors of daily life such as heart rate, autonomic nerve activity, serum electrolyte, ischemia, drug administration and the like (Makoto Arita, Morio Ito and Tetsunori Saikawa, eds, "QT Kankaku No Kiso To Rinsho (Introduction to and clinical applications of QT interval)," Igaku-Shoin Ltd. (1999)). There are many reports about studies on the relationship between the QT interval and the activity of the autonomic nerves, and both the activity of the sympathetic nerves and the activity of the parasympathetic nerves are known to be a regulator of the QT interval, but on the other hand, it is difficult to estimate the dynamic state of the activity of the autonomic nerves from only data on the QT interval. However, it has been demonstrated that there is a positive correlation between the QT interval and LF/HF, i.e., a measure of autonomic nerve activity balance (Y. Murakawa et al., Am. J. Cardiol., 69, 339-343 (1992)). This shows that, although it is difficult to estimate the dynamic state of the activity of the autonomic nerves from only the QT interval, a combination of the QT interval with heart rate variation power spectral analysis affords a clue to examine the balance of the activity of the autonomic nerves.

Accordingly, with a view to finding compounds having excellent autonomic nerve function regulating action, the present inventors have made extensive and intensive studies on the effect of ameliorating smoking-derived disturbance of the activity of the autonomic nerve attained by various foods, food ingredients, and traditional pharmaceutical products, using heart rate variability power spectral analysis and QT interval as a measure, and, as a result, have clarified that sesamin has the effect of inhibiting an increase in LF/HF value, the effect of alleviating the inhibition of the parasympathetic nerve activity, and the effect of inhibiting the prolongation of QT interval and, at the same time, can ameliorate the totally lowered activity of the autonomic nerves. That is, it has been found that sesamin can regulate the activity of the autonomic nerves.

Accordingly, the present invention provides foods and drinks having autonomic nerve function regulating activity, comprising sesamin and/or its analogue as an active ingredient, and a process for producing the same. More specifically, the present invention provides foods and drinks, which can prevent and ameliorate symptoms or diseases caused by the disturbance and lowering of the activity of the autonomic nerves, for example, shortness of breath, palpitation, stiffness in the shoulder and the neck, headache, gastrointestinal weakening, constipation, feeling of cold, vertigo, orthostatic dysregulation, anemia, susurrus aurium, itching of the ear, sense of uneasiness, anorexia, malaise, insomnia, arrhythmia, infertility, menstruational disorder, diabetes, symptoms in climacteric disturbance such as hot flash, glow, psychroesthesia, and acute sweating, silent myocardial infarction, asymptomatic hypoglycemia, and, further, ischemic heart diseases and sudden death, and a process for producing the same.

### EMBODIMENTS OF THE INVENTION

The present invention can be applied to sesamin, as well as to analogues of sesamin. In the present invention, sesamin and its analogue refer to, for example, dioxabicyclo[3.3.0]octane derivatives described in Japanese Unexamined Patent Publication (Kokai) No. 4(1992)-9331, and specific examples thereof include sesamin, sesaminol, episesamin, episesaminol, sesamorin, 2-(3,4-methylenedioxyphenyl)-6-(3-methoxy-4-hydroxyphenyl)-3,7-dioxabicyclo[3.3.0]octane, 2,6-bis(3-methoxy-4-hydroxyphenyl)-3,7-dioxabicyclo[3.3.0]octane, and 2-(3,4-methylenedioxyphenyl)-6-(3-methoxy-4-hydroxyphenoxy)-3,7-dioxabicyclo[3.3.0]octane. Further, glycosides of sesamin and its analogues may also be utilized. Furthermore, metabolites of sesamin and/or its analogues may also be utilized. The sesamin and its analogues according to the present invention may be produced, for example, by the method described in Japanese Unexamined Patent Publication (Kokai) No. 4(1992)-9331. They may be used in an extract form as such or, if necessary, after purification.

When the sesamin and/or its analogue according to the present invention are used as an autonomic nerve function regulating agent, the autonomic nerve function regulating agent may be in the form of health foods and drinks containing sesamin and/or its analogue and, further, food additives containing sesamin and/or its analogue. When autonomic nerve function regulating agent is used in the form of health foods and drinks, sesamin and/or its analogue may be formulated into, for example, dried foods, supplements, soft drinks, mineral water, and alcoholic drinks. The health foods and drinks, however, are not limited to these only.

When sesamin and/or its analogue according to the present invention are used as a therapeutic agent, the preparation may be a solid or a liquid. Examples of preparations include powders, tablets, pills, capsules, suppositories, granules, mixtures for internal use, suspensions, emulsions, and lotions. Pharmaceutically acceptable excipients may be added to the preparations according to the present invention. Excipients include diluents, perfumes, stabilizers, lubricants for suspensions, binders, preservatives, and disintegrators for tablets. They may be used alone or in a combination of two or more.

The amount of sesamin and/or its analogue (active ingredient) according to the present invention necessary for regulating smoking-derived transient disturbance of the activity of the autonomic nerves and for ameliorating the activity of the autonomic nerves is 10 mg per adult. However, it would be apparent that this effective amount may be varied depending upon factors, which cause the disturbance of or a lowering in the activity of the autonomic nerves, the type of disease, the characteristics, age, weight, and severity of symptom of the patient, and dosage form. Therefore, the effective amount of the sesamin and/or its analogue for developing autonomic nerve function regulating action is, for example, 0.5 to 100 mg, preferably 1 to 60 mg, more preferably 5 to 60 mg, per day. However, there is no particular upper limit on the effective amount.

When amelioration in smoking-derived transient disturbance of the activity of the autonomic nerves is contemplated, sesamin and/or its analogue as an active ingredient of the foods and drinks and pharmaceutical products according to the present invention are expected to develop the contemplated effect by ingestion before smoking, preferably 3 hr to just before smoking. On the other hand, when amelioration in disturbance or lowering of the activity of the autonomic nerve caused by chromic smoking or other causes is contemplated, the ingestion time zone and form are not particularly limited. Ingestion at various timings can develop the autonomic nerve function regulating activity.

### EXAMPLES

Although the present invention will be described in more detail with reference to the following examples, the invention is not intended to be limited thereby.

### Example 1. Regulating action against disturbance of activity of autonomic nerves caused by smoking

For nine male general university students, a test was carried out by a double blind, crossover study. Specifically, 3 soft capsules each containing 10 mg of sesamin and 3 placebo capsules were ingested, and the effect of the ingestion of the capsules on the activity of autonomic nerves was examined by a heart rate variability power spectrum method according to the method by Ue, H. et al. (Ue, H. et al., Ann. Noninvasive Electrocardiol., 5: 336-345 (2000)).

After the ingestion of each sample, the students smoked one cigarette over a period of 4 min. Just before and after smoking, 10 min after smoking, and 30 min after smoking, electrocardiograms were taken, and sampling was carried out at 1024 Hz using 14-bit A/D conversion. Heart rate variability obtained from the electrocardiograms were processed by fast Fourier transform for frequency analysis to provide a heart rate variability power spectrum. In order to separate and quantify the heart rate variability spectrum, spectral integral values of two frequency bands (0.04 to 0.15 Hz and 0.15 to 0.4 Hz) are mainly determined. The spectral integral value of the low frequency (0.04 to 0.15 Hz) band was used as a measure of the activity of the sympathetic nerves and the activity of the parasympathetic nerves, while the spectral integral value of the high frequency (0.15 to 0.4 Hz) band was used as a measure of the activity of the parasympathetic nerves. Further, the value obtained by dividing the spectral integral value of the low frequency band by the spectral integral value of the high frequency band, that is, LF/HF, was used as a measure of the balance between the activity of the sympathetic nerves and the activity of the parasympathetic nerves (Akselrod S et al., Science 213: 220-222 (1981) and Moritani T et al., J sportmed Sci. 7: 31-39 (1993)). Further, the heart depolarization-repolarizaton time was measured by the QT interval.

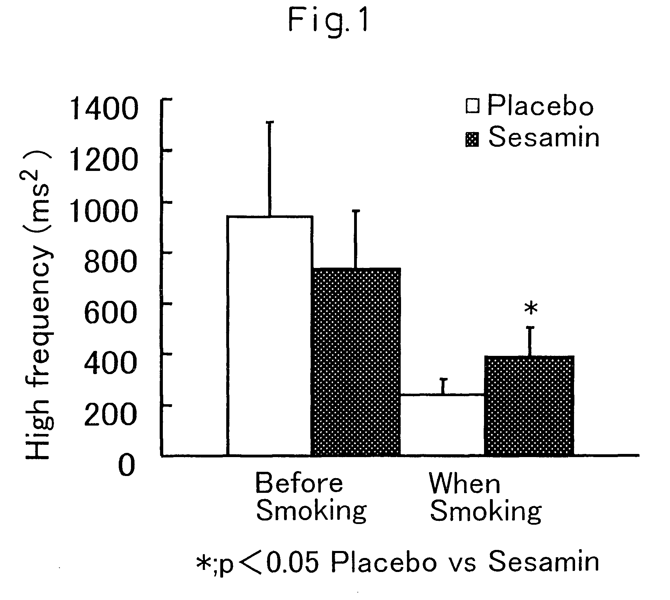

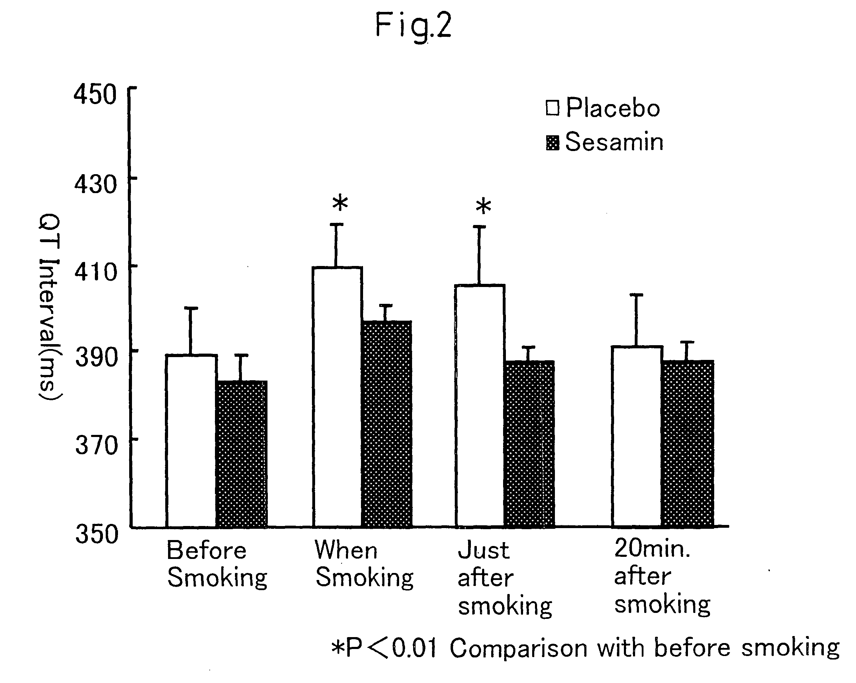

As a result, smoking caused a significant lowering in the activity of the parasympathetic nerves and a relative increase in the activity of the sympathetic nerves (for the placebo ingestion group, LF change: 852 before smoking → 1202 after smoking, HF change: 940 before smoking → 236 after smoking) and an increase in LF/HF (1.33 before smoking → 5.46 after smoking) which indicates that the activity of the sympathetic nerves is predominant. Even after 30 min of smoking, this change remained unchanged, and the LF/HF value did not return to the normal value before smoking (level of LF/HF change: just after smoking +4.13, 30 min after smoking +1.42). On the other hand, for the sesamin ingestion group, even at a time point just after smoking, autonomic nerve activity disturbance relaxation action was recognized, and, 30 min after smoking, the LF/HF value substantially returned to the value before smoking (level of LF/HF change: just after smoking +2.87, 30 min after smoking - 0.239). Further, it was found that, for the placebo ingestion group, smoking caused significantly lowered activity of the parasympathetic nerves, whereas, for the sesamin ingestion group, the lowering in the activity of the parasympathetic nerves can be significantly inhibited (p < 0.05) (Fig. 1). Furthermore, for the placebo ingestion group, smoking caused significant prolongation of QT interval (p < 0.01), whereas, for the sesamin ingestion group, no significant prolongation of QT interval was observed (Fig. 2).

The above experimental results demonstrate that sesamin can inhibit a smoking-derived significant lowering in the activity of the parasympathetic nerves and can ameliorate the disturbance of the activity of the autonomic nerves, that is, can demonstrate that sesamin has autonomic nerve function regulating action.

### Example 2. Autonomic nerve function ameliorating (activating) action

For 14 women who display symptoms of climacteric disorder, a test was carried out by double-blind parallel between-groups comparison. The subjects were randomly divided into two groups. Over a period of 4 weeks, 3 soft capsules each containing 10 mg of sesamin were ingested consecutively for one of the groups, and 3 placebo capsules were ingested consecutively for the other group. Before and after the ingestion of the sample, electrocardiograms were taken in a supine position, and the activity of the autonomic nerves was examined by heart rate variability power spectral analysis.

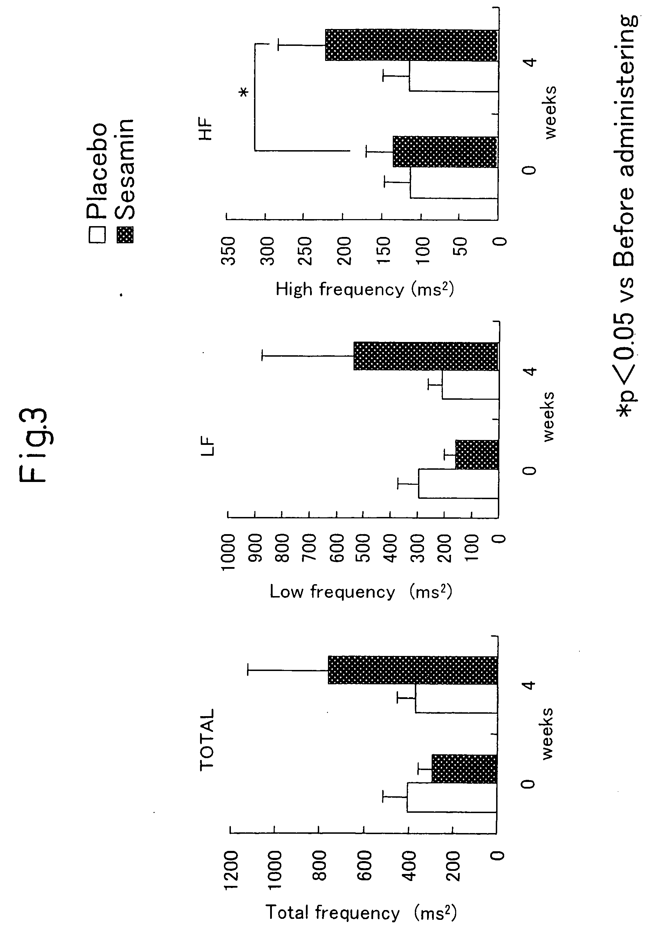

As a result, for the subjects of this test, the activity of the autonomic nerves before the start of the test was about 160 for LF and was about 140 for HF. Although the normal value of the activity of the autonomic nerves is not particularly specified, according to studies conducted by Yamasaki et al. (Yamasaki Y et al., Diabetes Res. 17: 73-80 (1991)), the autonomic nerve activity value of healthy people is 466 ± 332 for LF and 251 ± 151 for HF. In Example 1, for the male university students, the autonomic nerve activity value before smoking was 852 ± 238 for LF and was 940 ± 370 for HF. As compared with these autonomic nerve activity values (LF and HF), the subjects of this test who display symptoms of climacteric disorder had lower autonomic nerve activity values. For the placebo ingestion group, both the overall autonomic nerve activity and the sympathetic/parasympathetic nerve activity remained unchanged. On the other hand, for the sesamin ingestion group, the overall autonomic nerve activity and the sympathetic and parasympathetic nerve activities were increased, and, in particular, the parasympathetic nerve activity was significantly increased (Fig. 3). Thus, upon sesamin ingestion, an amelioration of the activity of the autonomic nerves particularly including an increase in the activity of the parasympathetic nerves could be realized.

The above experimental results show that sesamin can ameliorate totally lowered activity of the autonomic nerves.

### Preparation Example 1. Butter

| | |
|---|---|
| Sesamin | 1.2 g |
| Butter fat | 100 g |
| Tocopherol acetate | 1.2 g |

In agitating operation (churning) in a butter production process, 1.2 g of sesamin and, in addition, 1.2 g of tocopherol acetate were added to 100 g of butter fat from which butter milk had been removed, and a working operation was carried out to prepare an autonomic nerve function regulating butter containing the composition according to the present invention which had a homogeneous structure.

### Preparation Example 2. Granules

| | |
|---|---|
| Sesamin/episesamin mixture | 0.25 g |
| Tocopherol acetate | 0.25 g |
| Silicic anhydride | 20.5 g |
| Corn starch | 79 g |

The above ingredients were homogeneously mixed together. 100 ml of a solution of 10% hydroxypropylcellulose in ethanol was added thereto, and the mixture was kneaded by a conventional method, was extruded, and was dried to prepare granules.

### Preparation Example 3. Tablets

| | |
|---|---|
| Sesamin | 3.5 g |
| Tocopherol acetate | 0.5 g |
| Silicic anhydride | 20 g |
| Microcrystalline cellulose | 10 g |
| Magnesium stearate | 3 g |
| Lactose | 60 g |

The above ingredients were mixed together, and the mixture was tabletted by a single punch tabletting machine to prepare tablets having a diameter of 7 mm and a weight of 100 mg.

### Preparation Example 4. Capsules

| | |
|---|---|
| Gelatin | 70.0% |
| Glycerin | 22.9% |
| Methyl p-oxybenzoate | 0.15% |
| Propyl p-oxybenzoate | 0.51% |
| Water | q.s. |
| Total | 100% |

The following composition was filled into a soft capsule coating comprising the above ingredients by a conventional method to prepare soft capsules having a weight of 200 mg per capsule.

### Sesamin/episesamin (1 : 1)

| | |
|---|---|
| mixture | 10.8% |
| Wheat bead wax | 30% |
| α-Tocopherol | 20% |
| Palm oil | 10% |
| Wheat germ oil | q.s. |
| Total | 100% |

### Preparation Example 5. Health drink

| | |
|---|---|
| Taste agent: sodium DL-tartrate | 0.1 g |
| succinic acid | 0.009 g |
| Sweetening agent: liquid sugar | 800 g |
| Sour agent: citric acid | 12 g |
| Vitamin: vitamin C | 10 g |
| Sesamin | 1 g |
| Vitamin E | 30 g |
| Cyclodextrin | 5 g |
| Perfume | 15 ml |
| Potassium chloride | 1 g |
| Magnesium sulfate | 0.5 g |

The above ingredients were formulated, and water was added to bring the total volume to 10 liters. For this health drink, the unit dose is about 100 ml.

### INDUSTRIAL APPLICABILITY

The ingestion of sesamin and/or its analogue can inhibit an increase in the activity of sympathetic nerves and can inhibit a lowering in the activity of parasympathetic nerves. Further, the totally lowered activity of the autonomic nerves can be ameliorated. This autonomic nerve function regulating action is very useful for the prevention and alleviation of disturbance or a lowering in activity of the autonomic nerves and, at the same time, is very useful for various diseases caused when the activity of the sympathetic nerves is dominant or when the activity of the autonomic nerves is totally lowered.

## Claims

1. An agent having autonomic nerve function regulating action, comprising sesamin and/or episesamin as an active ingredient.

2. The agent having autonomic nerve function regulating action according to claim 1 for use in amelioration in lowered activity of the autonomic nerves, which comprises sesamin and/or episesamin as an active ingredient.

3. The agent having autonomic nerve function regulating action according to claim 1 for use in the prevention, amelioration, or alleviation in the disturbance or totally lowered activity of the autonomic nerves, and symptoms attributable thereto, which comprises sesamin and/or episesamin as an active ingredient.

4. The agent having autonomic nerve function regulating action according to any one of claims 1 to 3, wherein the autonomic nerve function regulating action is to inhibit an increase in the activity of sympathetic nerves.

5. The agent having autonomic nerve function regulating action according to any one of claims 1 to 3, wherein the autonomic nerve function regulating action is to increase the activity of parasympathetic nerves.

6. The agent having autonomic nerve function regulating action according to any one of claims 1 to 3, wherein the autonomic nerve function regulating action is to inhibit a lowering in the activity of parasympathetic nerves.

7. The agent having autonomic nerve function regulating action according to any one of claims 1 to 3, wherein the autonomic nerve function regulating action is to ameliorate lowered activity of the autonomic nerves.

8. The agent having autonomic nerve function regulating action according to any one of claims 1 to 7, which is a food or a drink.

9. The agent having autonomic nerve function regulating action according to any one of claims 1 to 7, which is a pharmaceutical product.

10. A method for using the agent having autonomic nerve function regulating action according to any one of claims 1 to 3, said method comprising the step of ingesting sesamin and/or episesamin to regulate the disturbance of the autonomic nerves or to ameliorate lowered activity of the autonomic nerves, whereby symptoms attributable to the disturbance or lowered activity of the autonomic nerves are prevented, ameliorated, or alleviated.

11. A method for inhibiting an increase in the activity of sympathetic nerves, comprising the step of ingesting sesamin and/or episesamin.

12. A method for increasing the activity of parasympathetic nerves, comprising the step of ingesting sesamin and/or episesamin.

13. A method for inhibiting a lowering in the activity of parasympathetic nerves, comprising the step of ingesting sesamin and/or episesamin.

14. A method for ameliorating totally lowered activity of the autonomic nerves, comprising the step of ingesting sesamin and/or episesamin.

15. A method for regulating the disturbance of the activity of autonomic nerves, comprising the step of ingesting sesamin and/or episesamin.

16. A method for ameliorating lowered activity of autonomic nerves, comprising the step of ingesting sesamin and/or episesamin.

17. An inhibitor for inhibiting the prolongation of QT interval, comprising sesamin and/or episesamin as an active ingredient.

18. A method for inhibiting the prolongation of QT interval, comprising the step of ingesting sesamin and/or episesamin.
